(19) Europäisches Patentamt
European Patent Office
Office européen des brevets

(11) EP 3 358 953 B1

(12) **FASCICULE DE BREVET EUROPEEN**

(45) Date de publication et mention de la délivrance du brevet:
**19.05.2021 Bulletin 2021/20**

(21) Numéro de dépôt: **16788180.4**

(22) Date de dépôt: **07.10.2016**

(51) Int Cl.:
*A61K 31/34* (2006.01) *A61K 31/215* (2006.01)
*A61K 31/395* (2006.01) *A01N 43/40* (2006.01)
*A01N 51/00* (2006.01) *A01N 53/00* (2006.01)
*A61P 33/02* (2006.01)

(86) Numéro de dépôt international:
**PCT/FR2016/052587**

(87) Numéro de publication internationale:
**WO 2017/060641 (13.04.2017 Gazette 2017/15)**

(54) **COMPOSITIONS VETERINAIRES POUR CONTROLER LES PHLEBOTOMES**
VETERINÄRE ZUSAMMENSETZUNGEN ZUR KONTROLLE VON PHLEBOTOMEN
VETERINARY COMPOSITIONS FOR THE CONTROL OF PHLEBOTOMES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.10.2015 FR 1559603**

(43) Date de publication de la demande:
**15.08.2018 Bulletin 2018/33**

(73) Titulaire: **Ceva Santé Animale**
**33500 Libourne (FR)**

(72) Inventeur: **VARLOUD, Marie**
**33500 Libourne (FR)**

(74) Mandataire: **Cabinet Becker et Associés**
**25, rue Louis le Grand**
**75002 Paris (FR)**

(56) Documents cités:
- **Anonymous: "Vectra 3D: Innovation antiparasitaire des laboratoires Ceva", Internet , 10 mars 2014 (2014-03-10), XP002758424, Extrait de l'Internet: URL:https://www.technidog.com [extrait le 2016-06-01]**
- **Anonymous: "CVMP assessment report for Vectra 3D", Internet , 10 octobre 2013 (2013-10-10), XP002758425, Extrait de l'Internet: URL:http://www.ema.europa.eu [extrait le 2016-06-01]**
- **Anonymous: "Dinotefuran + Pyriproxyfen + Permethrin for Dogs and Puppies", Internet , 8 septembre 2015 (2015-09-08), XP002758426, Extrait de l'Internet: URL:http://www.vetstreet.com [extrait le 2016-06-01]**
- **Anonymous: "Vectra 3D: 3 principios activos para une proteccion completa", Internet , février 2014 (2014-02), XP002758427, Vetmadrid 2014 - XXXI Congreso Anual de AMVAC, Jan-Feb 2014 Madrid, Spain Extrait de l'Internet: URL:http://www.amvac.es/docs/revistaCentro/ICV3.pdf [extrait le 2016-06-01]**
- **LIÉNARD E ET AL: "Efficacy of dinotefuran, permethrin and pyriproxyfen combination spot-on on dogs againstPhlebotomus perniciosusandCtenocephalides canis", PARASITOLOGY RESEARCH, SPRINGER VERLAG, BERLIN, DE, vol. 112, no. 11, 31 août 2013 (2013-08-31), pages 3799-3805, XP035356717, ISSN: 0932-0113, DOI: 10.1007/S00436-013-3568-Z [extrait le 2013-08-31]**
- **"Ceva VECTRA 3D", , 4 mars 2015 (2015-03-04), page 1 pp., XP054976572, Extrait de l'Internet: URL:https://www.youtube.com/watch?v=Fb9_Tg BLcKI [extrait le 2016-06-02]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 358 953 B1

Printed by Jouve, 75001 PARIS (FR)

## Description

DOMAINE TECHNIQUE DE L'INVENTION

La présente invention concerne l'utilisation de compositions dans le domaine vétérinaire et plus particulièrement dans le domaine de la parasitologie. L'invention concerne plus spécifiquement l'utilisation de compositions vétérinaires pour contrôler les phlébotomes et/ou pour contrôler la leishmaniose chez les mammifères non-humains, tels que les chats et les chiens.

ARRIERE-PLAN TECHNOLOGIQUE DE L'INVENTION

Les phlébotomes sont de petits insectes de l'ordre des Diptères, du sous-ordre des Nématocères et de la famille des Psychodidés. Ils sont largement répandus sur tout le pourtour méditerranéen et notamment sur une grande partie de l'Afrique et du Moyen-Orient, ainsi que dans plusieurs pays d'Amérique du Sud, tels que le Brésil.

Les phlébotomes sont le vecteur de la leishmaniose chez l'Homme et les mammifères, tels que les chiens et les chats ; et sont aussi des vecteurs de nombreux virus chez l'Homme. Leur rôle pathogène direct pouvant induire des réactions cutanées immédiates ou retardées est toutefois plus limité.

Plus particulièrement, *Phlebotomus perniciosus* est connu comme étant le principal vecteur de *Leishmania infantum* chez les humains et les chiens. Ainsi, différents traitements pour contrôler un tel parasite et/ou traiter et prévenir la leishmaniose ont été développés et mis en œuvre.

Par exemple, Danta-Torres (Vet. Parasitol., 2006, 141, 1-8) et Moreno et al. (PLoS One 6:e1683, 2012) ont développé des vaccins efficaces contre *L. infantum.* En particulier Moreno et al. ont montré que le vaccin LiESP/QA-21 était capable d'induire un profil de réponse cellulaire Th1 dans les trois semaines après la première vaccination.

EP 1 022 944 décrit l'utilisation d'un collier comprenant un composé de la famille des pyréthrinoïdes, en particulier la deltamétrine, pour protéger les chiens des morsures des phlébotomes.

Des formulations topiques ont également été développées pour contrôler les phlébotomes. Molina et al. (Veterinary Parasitology, 2012, 187, 529-533 ont notamment démontré que le produit ExSpot® (65% de perméthrine dans le monométhyl de propylène glycol) permettait de protéger les chiens contre les morsures de *P. perniciosus* pendant 22 jours après administration. Molina et al. (The Veterinary Record, August 12, 2006) ont également montré qu'une composition sous forme de spray comprenant de la perméthrine et du pyriproxyfène permettait d'obtenir un effet répulsif contre *P. perniciosus* pendant au moins 21 jours. Frenais et al. (Parasites & Vectors, 2014, 7:217) ont montré que le produit Activyl® Tick Plus (indoxacarb et perméthrine) à la dose minimale recommandée permettait d'obtenir une excellente efficacité répulsive contre *P. perniciosus* entre 2 et 14 jours ainsi qu'une efficacité significative pendant au moins 29 jours. Miro et al. (Veterinary Parasitology, 2007, 143, 375-379 ont montré un effet répulsif contre *P. perniciosus* pendant 21 jours chez les chiens d'une formulation de type « spot-on » comprenant la combinaison imidacloprid 10 % (poids/volume) / perméthrine 50 % (poids/volume). Enfin, Dumont et al. (Parasites & Vectors, 2015, 8:49) ont quant à eux démontré qu'une combinaison de fipronil avec la perméthrine présentait un effet répulsif significatif contre *P. perniciosus* dès son application chez les chiens, et ce pendant 4 semaines.

Tel que brièvement illustré ci-dessus, aucun traitement développé à ce jour utilisant des formulations topiques ne permet d'obtenir une efficacité répulsive et/ou insecticide contre *P. perniciosus* pendant une durée de plus de 4 semaines.

Ainsi, il persiste aujourd'hui dans le cadre de la lutte contre les phlébotomes et/ou du contrôle de la leishmaniose, un besoin de développer de nouvelles formulations présentant un effet répulsif et/ou insecticide contre les phlébotomes avec une efficacité prolongée.

RESUME DE L'INVENTION

Dans ce contexte, les inventeurs ont démontré de manière surprenante qu'une composition vétérinaire comprenant un composé de la famille des néonicotinoïdes et un composé de la famille des pyréthrinoïdes permet d'obtenir un contrôle prolongé des phlébotomes par rapport aux traitements déjà existants. En particulier, les inventeurs ont démontré qu'une telle composition permettait un contrôle efficace des phlébotomes au-delà de 4 semaines et en particulier pendant au moins 5 semaines, illustré notamment par une efficacité résiduelle répulsive contre *P. perniciosus* supérieure d'au moins 89 % au bout de 37 jours et d'au moins 74 % au bout de 44 jours.

La présente invention concerne donc l'utilisation d'une composition vétérinaire comprenant un composé de la famille des néonicotinoïdes et un composé de la famille des pyréthrinoïdes pour contrôler les phlébotomes chez les mammifères non-humains, ladite composition étant destinée à être administrée en une seule prise, à un temps T0 puis de nouveau à un temps T1, au moins 5 semaines après.

Dans un mode particulier selon l'invention, le ratio en poids du composé de la famille des pyréthrinoïdes sur

le composé de la famille des néonicotinoïdes est compris entre 4 et 15, préférentiellement entre 6 et 9, et encore plus préférentiellement entre 7 et 8.

Selon l'invention, le composé de la famille des néonicotinoïdes est le dinotéfurane. Selon l'invention, le composé de la famille des pyréthrinoïdes est la perméthrine.

Egalement selon l'invention, la composition comprend en outre un inhibiteur de la croissance des insectes.

Dans un mode préféré, le ratio en poids de l'inhibiteur de la croissance des insectes sur le composé de la famille des néonicotinoïdes est compris entre 0,05 et 0,12, préférentiellement entre 0,06 et 0,1, et encore plus préférentiellement entre 0,07 et 0,09.

Selon l'invention , l'inhibiteur de la croissance des insectes est le méthoprène ou le pyriproxyfène, de préférence le pyriproxyfène.

Toujours selon l'invention, la composition est destinée à être appliquée topiquement sur la peau du mammifère non-humain à un temps T0 puis de nouveau à un temps T1, au moins 5 semaines après, à rebrousse-poil sur la peau du mammifère non-humain.

Dans un mode encore préféré, la composition est destinée à être appliquée à rebrousse-poil en continu à partir de la base de la queue, le long de l'épine dorsale jusqu'au niveau des omoplates du mammifère non-humain.

Typiquement, le mammifère non-humain est un chien ou un chat, préférentiellement un chien. Particulièrement, les phlébotomes sont phlébotomes sont du genre *Phlebotomus, Sergentomyias ou Lutzomyia,* préférentiellement du genre *Phlebotomus.*

Un autre objet de la présente invention est une composition vétérinaire telle que décrite dans la présente demande, pour son utilisation dans le contrôle de la leishmaniose chez les mammifères non-humains, préférentiellement les chats et/ou les chiens, encore plus préférentiellement les chiens.

Un objet supplémentaire de l'invention concerne un kit comprenant un ou plusieurs compartiments, lesdits compartiments comprenant une combinaison du dinotéfurane, de la perméthrine et du pyriproxyfène pour une utilisation simultanée de ladite combinaison à un temps T0 puis de nouveau à un temps T1, au moins 5 semaines après , pour contrôler les phlébotomes et/ou contrôler la leishmaniose chez les mammifères non-humains, de préférence les chiens.

Dans un mode particulier, le kit comprend en outre une notice ou une fiche d'instructions concernant le mode d'administration et le mode opératoire de la combinaison pour contrôler les phlébotomes et/ou pour contrôler la leishmaniose chez les mammifères non-humains, de préférence les chiens.

DESCRIPTION DETAILLEE DE L'INVENTION

Composition

Les compositions vétérinaires de l'invention comprennent un composé de la famille des néonicotinoïdes et un composé de la famille des pyréthrinoïdes, pour contrôler les phlébotomes chez les mammifères non-humains, lesdits composés étant le dinotéfurane et la perméthrine, ainsi qu'un inhibiteur de la croissance des insectes qui est le pyriproxyfène.

Les composés de la famille des néonicotinoïdes sont chimiquement similaires à la nicotine. Ils correspondent à une classe d'insecticides agissant sur le système nerveux central des insectes en inhibant les récepteurs nicotiniques de l'acétylcholine, entraînant ainsi la paralysie et la mort de l'insecte parasite. Ils ont été introduits sur le marché dans les années 90 et sont particulièrement actifs contre les ectoparasites tels que les puces, les mouches et les poux. Des composés de la famille des néonicotinoïdes incluent sans limitation l'imidaclopride, le thiaméthoxam, la clothianidine, acétamipride, le thiaclopride, dinotéfurane, nitenpyram, imidaclothiz, huanyanglin, guadipyr, paichongding, cycloxaprid et leurs dérivés ou leurs sels pharmaceutiquement acceptables. Un composé de la famille des néonicotinoïdes préféré de l'invention est le dinotéfurane.

Au sens de la présente invention, le terme « dinotéfurane » comprend également ses dérivés ou analogues, ses métabolites et ses sels pharmaceutiquement acceptables.

Le dinotéfurane a été décrit par la compagnie Mitsui Toatsu Chemicals, Inc. Dans le brevet EP 0 649 845 et a été développé pour contrôler les insectes nuisibles. Le dinotéfurane dont le nom chimique est 2-méthyl-1-nitro-3-[(tétrahydro-3-furanyl) méthl] guanidine répond à la formule suivante :

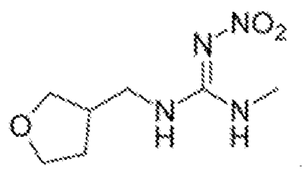

Les néonicotinoïdes et en particulier le dinotéfurane se métabolisent dans l'organisme des mammifères non-humains et prolongent ainsi leur durée d'action. Les principales voies métaboliques du dinotéfurane impliquent des réactions de N-déméthylation, de nitro réduction, de N-méthylène hydroxylation et de clivage aminé. Les métabolites du dinotéfurane comprennent les composés décrits par Simon-Delso et al. (Systemic insecticides (neonicotinoids and fipronil): trends, uses, mode of action and metabolites, Environ. Sci. Pollut. Res., 2015, 22:5-34) et par Ford KA and Casida JE (Unique and common metabolites of thiamethoxam, clothianidin, and dinotefuran in mice, Chem. Res. Toxicol., 2006, 19:1549-1556; Neonicotinoid metabolism: compounds, substituents, pathways, enzymes, organisms, and relevance, J. Agric. Food Chem., 2011, 59:2923-2931) et FAO dinotefuran (http://www.fao.org/fileadmin/templates/agphome/documents/ PestsPesticides /JMPR/ Evaluation12/Dinotefuran.pdf). En particulier, les metabolites du dinotéfurane incluent, sans limitation, N-desméthyl dinotéfurane (2-nitro-1-(tétrahydro-3-furylméthyl)guanidine), DIN-NNO, DIN-dm-NNO, DIN-NNH2, 1-méthyl-3-(tétrahydro-3-furylméthyl)guanidine, 3-(tétrahydro-3-furylméthyl)guanidine, 1-méthyl-3-(tétrahydro-3-furylméthyl)urée, 3-(tétrahydro-3-furylméthyl)ureé, 2-hydroxy-dinotéfurane, 4-hydroxy-dinotéfuran, 1,3-diazinane aminocarbinol, DIN-b (dérivé de DIN-dm), DIN-e (dérivé guanidine de DIN-a), DIN-f (dérivé guanidine de DIN-b), DIN-g (dérivé de DIN-5-OH), DIN-h (desméthyl-g), DIN-I (dérivé nitroso de DIN-g), DIN-j (dérivé nitroso de DIN-h), DIN-k (dérivé guanidine de DIN-h), tétrahydrofurane carboxaldéhyde (3-furfural), tétrahydrofurane alcool (3-furfuryl alcool), acide 3-tétrahydrofurane carboxylique, acide 4-hydroxy-3-tétrahydrofuranz carboxylique, tétrahydrofuran-3-yl-méthylamine, 1-[4-hydroxy-2-(hydroxyméthyl)butyl]-3-méthyl-2-nitroguanidine, et 3-hydroxy dinotéfurane.

Typiquement, les dérivés ou analogues du dinotéfurane comprennent tous les composés décrits dans le brevet EP 0 649 845. Plus particulièrement, les dérivés ou analogues du dinotéfurane comprennent, sans limitation, 1-[{(tétrahydro-3-furanyl)méthyl}amino]-1-méthylamino-2-nitroéthylene, 1-[{(tétrahydro-3-furanyl)méthyl}amino]-1-éthylamino-2-nitroéthylene, 1-[{(tétrahydro-3-furanyl)méthyl}amino]-1-diméthylamino-2-nitroéthylene, 1-[{(tétrahydro-3-furanyl)méthyl}amino]-1-(1-pyrrolidinyl)-2-nitroéthylene, 1-[N-{(tétrahydro-3-furanyl)méthyl}-N-méthylamino]-1-méthylamino-2-nitroéthylene, 1-[N-{(tétrahydro-3-furanyl)méthyl}-N-propylamino]-1-méthylamino-2-nitroéthylene, 1-[N-{(tétrahydro-3-furanyl)méthyl}-N-propylamino]-1-éthylamino-2-nitroéthylene, 1-{(tétrahydro-3-furanyl)méthyl}-2-nitro-3-methylguanidine, N-{(tétrahydro-3-furanyl)-méthyl}-N-(méthyl)nitroguanidine, 1-{(tétrahydro-3-furanyl)méthyl}-1-éthyl-2-nitro-3-méthylguanidine, N-(tétrahydro-3-furanyl)-méthyl-N'-cyano(méthylthio)formamidine, N-cyano-N'- { (tétrahydro-3-furanyl)méthyl} acétamidine, N-cyano-N'-{(tétrahydro-3-furanyl)méthyl}-N-méthylacetamidine, N-[4-{(2-méthyl)tétrahydrofuranyl}méthyl]-N'-méthyl-N"-nitroguanidine, 1-{(tétrahydro-3-furanyl)méthyl}-1,2-dicyclohexylcarbonyl-2-méthyl-3-nitroguanidine, 1-{(tétrahydro-3-furanyl)méthyl}-1,2-diethylcarbonyl-2-méthyl-3-nitroguanidine, 1-{(tétrahydro-3-furanyl)méthyl}-1,2-diméthoxycarbonyl-2-méthyl-3-nitroguanidine, et 1-{(tétrahydro-3-furanyl)méthyl} -1,2-dibenzoyl-2-méthyl-3-nitroguanidine.

Les composés de la famille des pyréthrinoïdes sont des composés agissant sur le système nerveux des insectes et perturbent le fonctionnement des neurones en interagissant avec les canaux sodiques. Des composés de la famille des pyréthrinoïdes incluent sans limitation l'éthofenprox, la perméthrine, la pralléthrine, la resméthrine, la sumithrine, l'alléthrine, l'alpha-cyperméthrine, la bifenthrine, la béta-cyperméthrine, la cyfluthrine, la cypermétrine, la deltaméthrine, la fluméthrine, l'esfenvalérate, la lamda-cyhalothrine, la zéta-cyperméthrine, et leurs dérivés ou leurs sels pharmaceutiquement acceptables.

Un composé préféré de la famille des pyréthrinoïdes est la perméthrine.

Selon l'invention, les compositions de l'invention comprennent en outre un inhibiteur de la croissance des insectes

Les inhibiteurs de la croissance des insectes sont des substances chimiques qui inhibent le cycle de vie des insectes. Parmi les inhibiteurs de la croissance des insectes, on peut citer sans limitation l'azadirachtine, l'hydroprène, le méthoprène, le pyriproxyfène, le triflumuron, et leurs dérivés ou leurs sels pharmaceutiquement acceptables.

Selon l'invention, l'inhibiteur de la croissance des insectes est le pyriproxyfène.

Par « sels pharmaceutiquement acceptables », on entend à la fois les sels organiques et inorganiques. Des exemples représentatifs de sels inorganiques comprennent les chlorhydrates, les bromhydrates, les iodates, les sulfonates et les phosphates. Des exemples représentatifs de sels organiques comprennent les formates, les acétates, les trichloroacétates, les proponiates, les benzoates, les cinnamates, les fumarates, les maléates, et les méthanesulfonates.

Dans un mode de réalisation particulier, la composition comprend un composé de la famille des néonicotinoïdes, qui est le dinotéfurane, et un composé de la famille des pyréthrinoïdes, qui est la perméthrine, dans des quantités en poids de telle sorte que le ratio en poids du composé de la famille des pyréthrinoïdes sur le composé de la famille des néonicotinoïdes soit compris entre 4 et 15, préférentiellement entre 6 et 9, et encore plus préférentiellement entre 7 et 8.

Dans un autre mode de réalisation particulier, la composition comprend un composé de la famille des néonicotinoïdes, qui est le dinotéfurane, un composé de la famille des pyréthrinoïdes, qui est la perméthrine, et un inhibiteur de la croissance des insectes, qui est le pyriproxyfène, dans des quantités en poids de telle sorte que le ratio en poids de l'inhibiteur de la croissance des insectes sur le composé de la famille des néonicotinoïdes soit compris entre 0,05 et 0,12, préférentiellement entre 0,06 et 0,1, et encore plus préférentiellement entre 0,07 et 0,09.

Dans un mode préféré de l'invention, la composition comprend environ 60 à 95 % de perméthrine, environ 6

à 22 % de dinotéfurane, et optionnellement environ 0,8 à 4 % de pyriproxyfène. Dans un mode plus préféré de l'invention, la composition comprend environ 70 à 90 % de perméthrine, environ 8 à 15 % de dinotéfurane, et optionnellement environ 0,9 à 1,5 % de pyriproxyfène. Selon ces modes préférés de l'invention, les pourcentages sont exprimés en poids par rapport au poids total des composés qui sont la perméthrine, le dinotéfurane et optionnellement le pyriproxyfène.

Dans un autre mode préféré de l'invention, la composition comprend :

- environ 20 à 50 %, préférentiellement environ 30 à 40 %, et encore plus préférentiellement environ 34 à 38 %, en poids de perméthrine,
- environ 1 à 20 %, préférentiellement environ 1 à 10 %, et encore plus préférentiellement environ 3 à 7 %, en poids de dinotéfurane, et optionnellement environ 0,1 à 2 %, préférentiellement environ de 0,1 à 1 %, et encore plus préférentiellement 0,2 à 0,6 % en poids de pyriproxyfène, par rapport au poids total de la composition.

Le terme environ sera compris par l'homme du métier et peut varier dans une certaine mesure selon le contexte dans lequel il est utilisé. Si certaines utilisations de ce terme ne sont pas claires pour l'homme du métier en fonction du contexte, « environ » signifie plus ou moins 20 %, de préférence plus ou moins 10 % du terme particulier.

Application

Par « mammifère non-humain », on entend tout mammifère non-humain susceptible d'être mordu ou piqué par les phlébotomes et de développer ainsi la leishmaniose ou d'être réservoir de cette maladie, c'est-à-dire souffrant déjà de la leishmaniose et pouvant contaminer un autre mammifère non-humain *via* les phlébotomes. Le mammifère non-humain peut notamment être un canidé tel qu'un chien, un renard, un coyote et un loup, ou un félidé tel qu'un chat, un lion, une panthère. Dans un mode de réalisation préféré, le mammifère non-humain appartient au groupe des animaux de compagnie et est préférentiellement un chien ou un chat, et encore plus préférentiellement un chien.

Par les termes « contrôle » et « contrôler », on entend, sans limitation, l'action de réduire, repousser (anti-engorgement), éradiquer, éliminer, tuer et prévenir les phlébotomes chez les mammifères non-humains. La notion de « prévenir » comprend aussi le fait de diminuer, de réduire ou d'éviter un repas sanguin des phlébotomes chez les mammifères non-humains ce qui se traduit par la réduction des risques d'infection des sujets environnants et contribue ainsi à limiter l'expansion de la leishmaniose.

L'invention est aussi relative à une méthode pour contrôler les phlébotomes chez les mammifères non-humains comprenant l'administration d'une quantité efficace en une seule prise, à un temps T0 puis de nouveau à un temps T1, au moins 5 semaines après d'une composition comprenant un composé de la famille des néonicotinoïdes, un composé de la famille des pyréthrinoïdes, et optionnellement, un inhibiteur de la croissance des insectes chez lesdits mammifères non-humains, lesdits composés étant le dinotéfurane, la perméthrine et le pyriproxyfène.

Dans le cadre de la présente invention, l'expression « quantité efficace » signifie la quantité du composé de la famille des néonicotinoïdes, du composé de la famille des pyréthrinoïdes, et optionnellement de l'inhibiteur de la croissance des insectes capable de causer un contrôle suffisant des phlébotomes. Il est accepté par l'homme de l'art et notamment par l'agence Européenne des Médicaments (EMA) qu'un contrôle suffisant est obtenu quand au moins 80 % des phlébotomes sont contrôlés.

L'effet répulsif des phlébotomes est évalué en utilisant la formule d'Abbott (Abbott, W.S. : A method of computing the effectiveness of an insecticide ; J. Econ. Entomol. ; 1925 ; 18 ; 265-267) calculant le pourcentage (%) correspondant à la différence entre le nombre de phlébotomes engorgés calculé chez un mammifère non-humain non-traité (groupe contrôle) et le nombre de phlébotomes engorgés calculé chez un mammifère non-humain traité (groupe traité) par rapport au nombre de phlébotomes engorgés calculé chez un mammifère non-humain non-traité, soit :

((Nombre de phlébotomes engorgés du groupe contrôle – Nombre de phlébotomes engorgés du groupe traité) / Nombre de phlébotomes engorgés du groupe contrôle) x 100.

Un phlébotome engorgé (ou nourri de sang) correspond à un phlébotome ayant déjà piqué ou mordu le mammifère non-humain. Les moyennes arithmétiques sont utilisées.

L'effet insecticide des phlébotomes est évalué en calculant le pourcentage (%) correspondant à la différence entre le nombre de phlébotomes observé chez un mammifère non-humain non-traité (groupe contrôle) et le nombre de phlébotomes observé chez un mammifère non-humain traité (groupe traité) par rapport au nombre de de phlébotomes observé chez un mammifère non-humain non-traité, soit :
((Nombre de phlébotomes du groupe contrôle - Nombre de phlébotomes du groupe traité) / Nombre de phlébotomes du groupe contrôle) x 100. Les moyennes arithmétiques sont utilisées.

Un objet de l'invention porte également sur une méthode pour contrôler au moins environ 80 % des phlébotomes

chez les mammifères non-humains comprenant l'administration d'une quantité efficace en une seule prise, à un temps T0 puis de nouveau à un temps T1, au moins 5 semaines après d'une composition comprenant un composé de la famille des néonicotinoïdes, un composé de la famille des pyréthrinoïdes, et un inhibiteur de la croissance des insectes chez lesdits mammifères non-humains, lesdits composés étant le dinotéfurane, la perméthrine et le pyriproxyfène.

Tel que brièvement décrit dans l'introduction, les phlébotomes sont reconnus comme étant des vecteurs de la leishmaniose. Les phlébotomes peuvent être notamment du genre *Phlebotomus, Sergentomyias ou Lutzomyia.*

Parmi les espèces appartenant au genre phlébotome, on peut citer *Phlebotomus ariasi, Phlebotomus balanicus, Phlebotomus intermedius, Phlebotomus longicuspis, Phlebotomus papatasi, Phlebotomus perniciosus,* et *Phlebotomus sergentif.* Sur les continents américains, les phlébotomes appartiennent au genre *Lutzomyia.* On peut notamment citer *Lutzomyia longipalpis.* Dans un mode préféré de l'invention, les phlébotomes sont du genre *Phlebotomus* et sont typiquement *Phlebotomus perniciosus.*

L'invention concerne donc également une composition vétérinaire comprenant un composé de la famille des néonicotinoïdes, un composé de la famille des pyréthrinoïdes, et un inhibiteur de la croissance des insectes, pour son utilisation dans le contrôle de la leishmaniose chez les mammifères non-humains, ladite composition étant destinée à être administrée en une seule prise, à un temps T0 puis de nouveau à un temps T1, au moins 5 semaines après, lesdits composés étant le dinotéfurane, la perméthrine et le pyriproxyfène.

L'invention concerne en outre l'utilisation d'une composition vétérinaire comprenant un composé de la famille des néonicotinoïdes, un composé de la famille des pyréthrinoïdes, et un inhibiteur de la croissance des insectes, dans la fabrication d'un médicament pour contrôler la leishmaniose chez les mammifères non-humains, ladite composition étant destinée à être administrée en une seule prise, à un temps T0 puis de nouveau à un temps T1, au moins 5 semaines après, lesdits composés étant le dinotéfurane, la perméthrine et le pyriproxyfène. semaine

L'invention concerne également une méthode pour contrôler la leishmaniose chez des mammifères non-humains comprenant l'administration d'une quantité efficace d'une composition comprenant un composé de la famille des néonicotinoïdes, un composé de la famille des pyréthrinoïdes, et un inhibiteur de la croissance des insectes chez les mammifères non-humains souffrant ou non de leishmaniose, ladite composition étant destinée à être administrée en une seule prise, à un temps T0 puis de nouveau à un temps T1, au moins 5 semaines après, lesdits composés étant le dinotéfurane, la perméthrine et le pyriproxyfène.

Dans le contexte de l'invention, les expressions « contrôle de la leishmaniose », « traitement de la leishmaniose », et « prévention de la leishmaniose » désignent la protection du mammifère non-humain contre la leishmaniose, c'est-à-dire la protection du mammifère non-humain par le contrôle des phlébotomes. Selon un mode particulier, ces expressions incluent le traitement préventif du mammifère non-humain contre la leishmaniose.

Selon un premier aspect, un traitement préventif désigne un traitement réalisé avant que le mammifère non-humain ait été exposé ou ait été en contact avec l'agent causatif de la leishmaniose, en l'occurrence les phlébotomes. Un traitement préventif réduit donc les risques pour le mammifère non-humain de développer la leishmaniose.

Selon un second aspect, un traitement préventif désigne aussi un traitement réalisé chez un mammifère non-humain souffrant de leishmaniose. Le traitement réalisé chez un sujet malade permet de contrôler les phlébotomes dans son environnement et de réduire les risques d'infection chez les sujets sains environnants, contribuant ainsi à limiter l'expansion de la leishmaniose.

Selon l'invention, la composition est destinée à être administrée en une seule prise, à un temps T0 puis est de nouveau administré à un temps T1, au moins 5 semaines après. Particulièrement, le traitement peut être répété toutes les 5 semaines, ou de préférence pendant 10 semaines, 15 semaines, 20 semaines, 25 semaines, 50 semaines, 2 ans, 5 ans, et encore plus préférentiellement jusqu'à la mort du mammifère non-humain.

Selon l'invention, les compositions telles que définies ci-dessus sont destinées à être appliquées topiquement sur la peau du mammifère non-humain. Typiquement, les compositions utilisées dans la présente invention peuvent être sous forme de solutions, d'émulsions ou de gels. Les compositions de type « spot-on » ou « line-on » sont particulièrement bien adaptées pour une application topique.

Selon l'invention, les compositions sont appliquées à rebrousse-poil sur la peau du mammifère non-humain. On entend par une application à rebrousse-poil, une application dans le sens contraire de l'implantation des poils sur la peau du mammifère non-humain. Pour les mammifères non-humains concernés par l'invention, l'application à rebrousse-poil s'effectue généralement dans le sens partant de la base de la queue vers les omoplates du mammifère non-humain.

Avantageusement, les compositions vétérinaires utilisées dans l'invention sont appliquées à rebrousse-poil et en continu. L'application en continu de la composition est réalisée en maintenant en contact la composition avec la peau de l'animal, et ce jusqu'à ce que l'intégralité de la dose souhaitée de la composition soit administrée par vois externe sur la peau du mammifère non-humain. La notion « en continu » peut également être définie comme une succession infinie de points (spots).

L'application en continu de la composition peut être réalisée selon une trajectoire rectiligne. Dans un mode de réalisation préféré, les compositions utilisées sont appliquées à rebrousse-poil en continu à partir de la base de la queue,

le long de l'épine dorsale jusqu'au niveau des omoplates du mammifère non-humain. Particulièrement, l'utilisateur maintient l'animal avec une de ses mains contre lui et applique la composition à rebrousse-poil en continu à l'aide de son autre main à partir de la base de la queue, le long de l'épine dorsale jusqu'au niveau des omoplates du mammifère non-humain.

Les compositions utilisées dans l'invention sont préférentiellement formulées sous forme de dose unitaire adaptée au poids et/ou à la taille du mammifère non-humain, l'intégralité de la dose étant appliquée sur le mammifère non-humain. Typiquement, les doses de composition varient de 0,1 à 100 mL, préférentiellement de 0,5 à 20 mL, et encore plus préférentiellement de 0,5 à 10 mL. Lorsque les mammifères non-humains concernés par l'invention sont des chiens et des chats, les doses de composition varient typiquement de 0,5 à 10 mL. Selon certains modes de réalisation particuliers, les compositions sont appliquées à une dose comprise entre environ 0,05 et 0,6 mL/kg de poids corporel du mammifère non-humain.

Selon un autre aspect de l'invention, le composé de la famille des néonicotinoïdes, le composé de la famille des pyréthrinoïdes et l'inhibiteur de la croissance des insectes tels que définis précédemment et incluant les modes de réalisations préférées sont dans des compositions distinctes les unes des autres.

L'invention porte donc sur l'utilisation d'une combinaison destinée à être administrée en une seule prise, à un temps T0 puis de nouveau à un temps T1, au moins 5 semaines après et comprenant un composé de la famille des néonicotinoïdes, un composé de la famille des pyréthrinoïdes et, un inhibiteur de la croissance des insectes pour contrôler les phlébotomes chez les mammifères non-humains, lesdits composés étant le dinotéfurane, la perméthrine et le pyriproxyfène. . Un objet supplémentaire de l'invention porte sur une telle combinaison pour son utilisation dans le contrôle de la leishmaniose chez les mammifères non-humains.

Ainsi, la présente invention fournit également un kit comprenant un ou plusieurs compartiments, lesdits compartiments comprenant une combinaison d'un composé de la famille des néonicotinoïdes, un composé de la famille des pyréthrinoïdes, et un inhibiteur de la croissance des insectes, lesdits composés étant le dinotéfurane, la perméthrine et le pyriproxyfène, pour une utilisation simultanée de ladite combinaison à un temps T0 puis de nouveau à un temps T1, au moins 5 semaines après pour contrôler les phlébotomes et/ou pour contrôler la leishmaniose chez les mammifères non-humains, de préférence les chiens.

L'expression « utilisation simultanée » signifie que les composés formant la combinaison telle que définie ci-dessus sont administrés en même temps chez les mammifères non-humains.

Selon un mode de réalisation spécifique, le kit comprend en outre une notice ou une fiche d'instruction et le mode opératoire de la combinaison pour contrôler les phlébotomes et/ou pour contrôler la leishmaniose chez les mammifères non-humains, de préférence les chiens.

Un produit pour mettre en œuvre l'utilisation des compositions de l'invention pour contrôler les phlébotomes et/ou pour contrôler la leishmaniose chez les mammifères non-humains peut être notamment le produit comprenant la combinaison dinotéfurane-perméthrine-pyriproxyfène tel que celui commercialisé sous le nom Vectra®3D par Ceva Santé Animale.

D'autres aspects et avantages de l'invention apparaîtront à la lecture des exemples qui suivent, qui doivent être considérés comme illustratifs et non limitatifs.

## EXEMPLES

Exemple 1

Matériel et méthodes.

Seize chiens de race « Beagle » ont été répartis en 2 groupes homogènes selon leur capacité à être piqués par des phlébotomes (Jour -7). Le premier groupe (n=8, 12.5 ± 1.4 kg poids corporel moyen) correspond au groupe contrôle. 3,6 mL de Vectra®3D (composition de l'invention comprenant 4.95 % en poids de dinotéfurane, 36,08 % en poids de perméthrine, et 0,44 % en poids de pyriproxyfène, par rapport au poids total de la composition) sont administrés au second groupe (n=8, 11.6 ± 0.7 kg poids corporel moyen) au jour 0. Chaque chien est ensuite exposé pendant 1 heure, dans le noir, à 85±10 femelles adultes non engorgées et 10-20 males de *Phlebotomus perniciosus* à J-7, J2, J9, J16, J23, J30, J37, J44. Les phlébotomes utilisés étaient à jeun. Les phlébotomes ont été collectés une heure après chaque exposition. Tous les phlébotomes morts ou vivants ont été gelés et les femelles ont été classées comme étant engorgées ou non engorgées par un examen visuel à l'aide d'un stéréomicroscope. L'effet répulsif a été calculé en utilisant la formule d'Abbott et en considérant toutes les femelles engorgées comme un échec. L'effet insecticide a été calculé en utilisant la formule d'Abbott et en considérant toutes les femelles vivantes comme un échec. Les effets répulsif et insecticide de Vectra®3D ont été évalués à J2, J9, J16, J23, J30, J37, J44 par analyse de variance et par des tests non-paramétriques (Kriuskal-Wallis).

Résultats

Les résultats de l'étude sont présentés ci-dessous dans le tableau 1 et sont comparés avec différents produits commercialisés dans le contrôle des phlébotomes.

**Tableau 1**

| Produit | | J+2 S0 | J+9 S1 | J+16 S2 | J+23 S3 | J+30 S4 | J+37 S5 | J+44 S6 |
|---|---|---|---|---|---|---|---|---|
| **Vectra®3D** (Dinotéfuran - Perméthrine-pyriproxifène) | Efficacité répulsive (%) | 98 | 97 | 99 | 95 | 92 | **89** | **74** |
| | Efficacité insecticide (%) | 47 | 45 | 30 | 24 | 19 | 17 | 8 |
| [1]Exspot™ (Perméthrine) | Efficacité répulsive (%) | 99 | 93 | 87 | 68 | 61 | 57 | 18 |
| | Efficacité insecticide (%) | 98 | 80 | 43 | 32 | 24 | 6 | 3 |
| [2]Duowin Spray (perméthrine - pyriproxifène) | Efficacité répulsive (%) | - | 99 | 81 | 71 | 47 | 45 | 38 |
| | Efficacité insecticide (%) | - | 30 | 32 | 7 | 1 | 3 | 1 |
| [3]Activyl® Tick Plus (indoxacarb - perméthrine) | Efficacité répulsive (%) | 99 | 98 | 96 | 88 | 84 | 60 | 38 |
| | Efficacité insecticide (%) | 32 | 27 | 9 | 0 | 4 | 1 | - |
| [4]Advantix® (imidacloprid - perméthrine) | Efficacité répulsive (%) | 98 | 96 | 96 | 93 | 74 | 57 | 42 |
| | Efficacité insecticide (%) | 53 | 49 | 15 | 13 | 3 | 2 | - |
| [5]Frontline TriAct® (fipronil - perméthrine) | Efficacité répulsive (%) | 99 | 99 | 99 | 93 | 81 | 53 | 47 |
| | Efficacité répulsive (%) | 98 | 99 | 98 | 99 | 66 | 27 | - |

[1]Molina et al., Veterinary Parasitology, 2012, 187, 529-533.
[2]Molina et al., Veterinary Record, 2006, 159, 206-209.
[3]Fresnais et al., Parasites & Vectors, 2014, 7:217.
[4]Miro et al., Veterinary Parasitology, 2007, 143, 375-379.
[5]Dumont et al., Parasites & Vectors, 2015, 8 :49.
J = Jour
S = Semaine

Les résultats montrent que la composition de l'invention comprenant le dinotéfurane et la perméthrine présente une efficacité répulsive contre les phlébotomes supérieure à 80 % au bout de cinq semaines alors que les produits de l'art antérieur ne permettent d'obtenir une protection acceptable (c'est-à-dire une efficacité répulsive supérieure à 80 %) que pendant une période maximale de 4 semaines.

Les inventeurs ont donc démontré de manière surprenante que les compositions de l'invention permettaient de contrôler les phlébotomes chez les mammifères non-humains avec une efficacité prolongée, supérieure ou égale à 5 semaines, comparé aux traitements déjà existants.

Exemple 2

Effet d'un produit comprenant du dinotefuran, de la perméthrine et du pyriproxyfène contre *L. longipalpis* chez des chiens expérimentalement infestés.

Leishmania (Kinetoplastida, trypanosomatidae) sont des parasites protozoaires d'une grande importance médicale et vétérinaire, qui sont transmis à un hôte par le phlébotome du genre Phlebotomus sur le continent Européen et Lutzomyia sur le continent Américain.

Cette étude a été conçue pour déterminer l'efficacité insecticide (répulsivité) du produit Vectra® 3D (dinotefuran, perméthrine et pyriproxyfène) contre *L. longipalpis* chez les chiens expérimentalement infestés. Douze chiens adultes ont été attribués à un groupe traité et un groupe de contrôle basé sur le taux d'alimentation durant le pré-traitement et le sexe. Les chiens du groupe traité ont été traités par Vectra® 3D localement le jour 0. Tous les animaux ont été exposés sous sédation et pendant 1 heure à des phlébotomes adultes à jeun (5 - 200 mâles et femelles), les jours J+1, J+7, J+14, D+21, J+28, J+35 et J+42.

Après l'exposition aux phlébotomes, les chiens ont été retirés de la chambre d'exposition et les phlébotomes ont été recueillis, classés comme vivants ou morts et stockés.
Les phlébotomes vivants ont été incubés pendant 24 heures après l'exposition pour l'évaluation de la survie. Tous les insectes ont été classés comme mâle ou femelle et remplis de sang ou non-nourris. L'efficacité insecticide (répulsivité) a été calculée en utilisant la formule Abbott et le nombre moyen de femelles remplies de sang dans les groupes traités et non traités. Le nombre de phlébotomes nourris ont été comparés par un test de Student, en utilisant le logiciel statistique, SigmaPlot12.

Le nombre moyen de femelles en vie et remplies de sang observées pendant toute la période expérimentale était de 51,96, résultat qui démontre le succès de la méthodologie sur l'exposition des phlébotomes. Au cours de l'analyse statistique, on a montré une différence significative (p <0,05) pour tous les essais lorsque l'on compare le groupe traité au groupe témoin (Tableau 2).

Tableau 2. Nombre moyen de femelles en vie et remplies de sang sur les chiens soumis à la présente étude aux jours : J+1, J+7, J+14, J+21, J+28, J+35, et J+42.

**Tableau 2**

| **Jours** | **Moyenne** | | **p Valeur** | **Pertinence statistique (significative =Sig.)** |
|---|---|---|---|---|
| | **Traité** | **Contrôle** | | |
| **J+1** | 0,83 | 45,66 | 0,002 | Sig. |
| **J+7** | 0,33 | 47,33 | 0,002 | Sig. |
| **J+14** | 3,16 | 57,33 | 0,002 | Sig. |
| **J+21** | 5,66 | 54,83 | 0,002 | Sig. |
| **J+28** | 14,33 | 54,66 | <0,001 | Sig. |
| **J+35** | 12,85 | 56,27 | 0,002 | Sig. |
| **J+42** | 29,06 | 55,75 | 0,005 | Sig. |

La différence entre les groupes était significative à tous les points temporels (p <0,05). L'efficacité moyenne (87,45%) au cours de toute la période expérimentale était suffisante (> 80%). Ces résultats démontrent que Vectra® 3D fournit un effet insecticide / répulsif efficace contre *L. longipalpis* pendant 5 semaines (J+35), voire au-delà (J+42).

## Revendications

1. Composition vétérinaire comprenant du dinotéfurane, de la perméthrine, et du pyriproxyfène pour son utilisation pour contrôler les phlébotomes chez les mammifères non-humains, **caractérisée en ce que** la composition est destinée à être administrée en une seule prise, à un temps T0 puis de nouveau à un temps T1, au moins 5 semaines après, ladite composition étant destinée à être appliquée topiquement et à rebrousse-poil sur la peau du mammifère non-humain.

2. Composition vétérinaire pour son utilisation selon la revendication 1, **caractérisée en ce que** le ratio en poids de la perméthrine sur le dinotéfurane est compris entre 4 et 15.

3. Composition vétérinaire pour son utilisation selon la revendication 1 ou 2, **caractérisée en ce que** le ratio en poids de la perméthrine sur le dinotéfurane est compris entre 6 et 9.

4. Composition vétérinaire pour son utilisation selon l'une quelconque des revendications 1 à 3, **caractérisée en ce que** le ratio en poids de la perméthrine sur le dinotéfurane est compris entre 7 et 8.

5. Composition vétérinaire pour son utilisation selon l'une quelconques des revendications 1 à 4, **caractérisée en ce que** le ratio en poids du pyriproxyfène sur le dinotéfurane est compris entre 0,05 et 0,12.

6. Composition vétérinaire pour son utilisation selon l'une quelconques des revendications 1 à 5, **caractérisée en ce que** le ratio en poids du pyriproxyfène sur le dinotéfurane est compris entre 0,06 et 0,1.

7. Composition vétérinaire pour son utilisation selon l'une quelconques des revendications 1 à 6, **caractérisée en ce que** le ratio en poids du pyriproxyfène sur le dinotéfurane est compris entre 0,07 et 0,09.

8. Composition vétérinaire pour son utilisation selon l'une quelconque des revendications 1 à 7, **caractérisée en ce que** la composition est destinée à être appliquée à rebrousse-poil en continu à partir de la base de la queue, le long de l'épine dorsale jusqu'au niveau des omoplates du mammifère non-humain.

9. Composition vétérinaire pour son utilisation selon l'une quelconque des revendications 1 à 8, **caractérisée en ce que** le mammifère non-humain est un chien ou un chat, de préférence un chien.

10. Composition vétérinaire pour son utilisation selon l'une quelconque des revendications 1 à 9, **caractérisée en ce que** les phlébotomes sont du genre *Phlebotomus, Sergentomyias* ou *Lutzomyia,* préférentiellement du genre *Phlebotomus.*

11. Composition vétérinaire telle que définie selon l'une quelconques des revendications 1 à 8, pour son utilisation dans le contrôle de la leishmaniose chez les mammifères non-humains, préférentiellement les chats et/ou les chiens, encore plus préférentiellement les chiens.

## Patentansprüche

1. Veterinäre Zusammensetzung, umfassend Dinotefuran, Permethrin und Pyriproxyfen zur Verwendung zur Kontrolle von Phlebotomen bei nichthumanen Säugetieren, **dadurch gekennzeichnet, dass** die Zusammensetzung in einer Einzeldosis zu einer Zeit T0 und dann wieder zu einer Zeit T1 wenigstens 5 Wochen danach verabreicht werden soll, wobei die Zusammensetzung topisch und gegen den Fellstrich auf die Haut des nichthumanen Säugetiers appliziert werden soll.

2. Veterinäre Zusammensetzung zur Verwendung gemäß Anspruch 1, **dadurch gekennzeichnet, dass** das Gewichtsverhältnis von Permethrin zu Dinotefuran zwischen 4 und 15 beträgt.

3. Veterinäre Zusammensetzung zur Verwendung gemäß Anspruch 1 oder 2, **dadurch gekennzeichnet, dass** das Gewichtsverhältnis von Permethrin zu Dinotefuran zwischen 6 und 9 beträgt.

4. Veterinäre Zusammensetzung zur Verwendung gemäß einem der Ansprüche 1 bis 3, **dadurch gekennzeichnet, dass** das Gewichtsverhältnis von Permethrin zu Dinotefuran zwischen 7 und 8 beträgt.

5. Veterinäre Zusammensetzung zur Verwendung gemäß einem der Ansprüche 1 bis 4, **dadurch gekennzeichnet, dass** das Gewichtsverhältnis von Pyriproxyfen zu Dinotefuran zwischen 0,05 und 0,12 beträgt.

6. Veterinäre Zusammensetzung zur Verwendung gemäß einem der Ansprüche 1 bis 5, **dadurch gekennzeichnet, dass** das Gewichtsverhältnis von Pyriproxyfen zu Dinotefuran zwischen 0,06 und 0,1 beträgt.

7. Veterinäre Zusammensetzung zur Verwendung gemäß einem der Ansprüche 1 bis 6, **dadurch gekennzeichnet, dass** das Gewichtsverhältnis von Pyriproxyfen zu Dinotefuran zwischen 0,07 und 0,09 beträgt.

8. Veterinäre Zusammensetzung zur Verwendung gemäß einem der Ansprüche 1 bis 7, **dadurch gekennzeichnet, dass** die Zusammensetzung gegen den Fellstrich fortlaufend von der Schwanzbasis entlang des Rückgrats bis auf Höhe der Schulterblätter des nichthumanen Säugetiers appliziert werden soll.

9. Veterinäre Zusammensetzung zur Verwendung gemäß einem der Ansprüche 1 bis 8, **dadurch gekennzeichnet, dass** das nichthumane Säugetier ein Hund oder eine Katze, vorzugsweise ein Hund ist.

10. Veterinäre Zusammensetzung zur Verwendung gemäß einem der Ansprüche 1 bis 9, **dadurch gekennzeichnet, dass** die Phlebotome zur Gattung *Phlebotomus, Sergentomyias* oder *Lutzomyia,* vorzugsweise zur Gattung *Phlebotomus* gehören.

11. Veterinäre Zusammensetzung, definiert gemäß einem der Ansprüche 1 bis 8, zur Verwendung bei der Kontrolle der Leishmaniose bei nichthumanen Säugetieren, vorzugsweise bei Katzen und/oder Hunden, insbesondere bei Hunden.

## Claims

1. A veterinary composition comprising dinotefuran, permethrin, and pyriproxyfen, for use for sandfly control in non-human mammals, wherein the composition is intended to be administered in a single take, at a time T0 and then again at a time T1, at least 5 weeks later, said composition being intended to be topically applied to the skin of the non-human mammal, against the direction of the coat.

2. The veterinary composition for use according to claim 1, wherein the weight ratio of permethrin to dinotefuran is between 4 and 15.

3. The veterinary composition for use according to claim 1 or 2, wherein the weight ratio of permethrin to dinotefuran is between 6 and 9.

4. The veterinary composition for use according to any one of claims 1 to 3, wherein the weight ratio of permethrin to dinotefuran is between 7 and 8.

5. The veterinary composition for use according to any one of claims 1 to 4, wherein the weight ratio of pyriproxyfen to dinotefuran is between 0.05 and 0.12.

6. The veterinary composition for use according to any one of claims 1 to 5, wherein the weight ratio of pyriproxyfen to dinotefuran is between 0.06 and 0.1.

7. The veterinary composition for use according to any one of claims 1 to 6, wherein the weight ratio of pyriproxyfen to dinotefuran is between 0.07 and 0.09.

8. The veterinary composition for use according to any one of claims 1 to 7, wherein the composition is intended to be applied continuously against the direction of the coat starting from the base of the tail, along the dorsal spine up to the level of the shoulder blades of the non-human mammal.

9. The veterinary composition for use according to any one of claims 1 to 8, wherein the non-human mammal is a dog or a cat, preferably a dog.

10. The veterinary composition for use according to any one of claims 1 to 9, wherein the sandflies are of the *Phlebotomus,*

*Sergentomyias* or *Lutzomyia* genus, preferentially of the *Phlebotomus* genus.

**11.** The veterinary composition as defined in any one of claims 1 to 8, for use for the control of leishmaniosis in non-human mammals, preferentially dogs and/or cats, even more preferentially dogs.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1022944 A
- EP 0649845 A


### Littérature non-brevet citée dans la description


- **DANTA-TORRES.** *Vet. Parasitol.,* 2006, vol. 141, 1-8
- **MORENO et al.** *PLoS One,* 2012, vol. 6, e1683
- **MOLINA et al.** *Veterinary Parasitology,* 2012, vol. 187, 529-533
- **MOLINA et al.** *The Veterinary Record,* 12 Août 2006
- **FRENAIS et al.** *Parasites & Vectors,* 2014, vol. 7, 217
- **MIRO et al.** *Veterinary Parasitology,* 2007, vol. 143, 375-379
- **DUMONT et al.** *Parasites & Vectors,* 2015, vol. 8, 49
- **SIMON-DELSO et al.** Systemic insecticides (neonicotinoids and fipronil): trends, uses, mode of action and metabolites, Environ. *Sci. Pollut. Res.,* 2015, vol. 22, 5-34
- **FORD KA ; CASIDA JE.** Unique and common metabolites of thiamethoxam, clothianidin, and dinotefuran in mice. *Chem. Res. Toxicol.,* 2006, vol. 19, 1549-1556
- *J. Agric. Food Chem.,* 2011, vol. 59, 2923-2931
- **ABBOTT, W.S.** A method of computing the effectiveness of an insecticide. *J. Econ. Entomol.,* 1925, vol. 18, 265-267
- **MOLINA et al.** *Veterinary Record,* 2006, vol. 159, 206-209
- **FRESNAIS et al.** *Parasites & Vectors,* 2014, vol. 7, 217